# EUROPÄISCHE PATENTSCHRIFT

(11) **EP 3 632 354 B1**
(45) Veröffentlichungstag und Bekanntmachung des Hinweises auf die Patenterteilung: **31.08.2022**
(21) Anmeldenummer: 18198761.1
(22) Anmeldetag: 05.10.2018
(51) Int. Cl.: A61B 17/29, A61B 18/14

(54) **GEWEBEZANGE**
TISSUE FORCEPS
PINCE À TISSU

(43) Veröffentlichungstag der Anmeldung: 08.04.2020
(73) Patentinhaber: Erbe Elektromedizin GmbH, 72072 Tübingen (DE)
(72) Erfinder: Löser, David, 72108 Rottenburg am Neckar (DE)
(74) Vertreter: Rüger Abel Patentanwälte PartGmbB

(56) Entgegenhaltungen:
- DE-A1-102004 013 530
- DE-U1-202007 009 165

## Beschreibung

Die Erfindung betrifft eine Gewebezange oder dergleichen Instrument, insbesondere in Ausführung als laparoskopisches Instrument.

Aus der EP 2 959 854 A1 ist eine als laparoskopisches Instrument ausgebildete Gewebezange bekannt, die am distalen Ende eines länglichen Schafts ein Werkzeug mit zwei Branchen aufweist, zwischen denen Gewebe zu fassen, zu koagulieren und gegebenenfalls zu trennen ist. Während eine der Branchen mit dem Schaft starr verbunden ist, ist die andere Branche relativ dazu schwenkbar gelagert. Zur Lagerung der Branche dient ein Querstift, der sowohl einen das proximale Ende der beweglichen Branche aufnehmenden Sockel wie auch das proximale Ende der beweglichen Branche durchsetzt. Wenn an dem Instrument zusätzlich zu den Branchen noch ein Messer vorzusehen ist, das bei Nichtgebrauch in zurückgezogenen Zustand etwa auf Höhe des Branchengelenks steht und das bei geschlossenen Branchen zum Durchtrennen gefassten Gewebes in distaler Richtung vorschiebbar sein muss, muss dieses Messer durch das Branchengelenk oder an diesem vorbeigeführt werden. Dazu sieht die DE 20 2007 009 165 U1 einen Lagerzapfen vor, der in seinem mittleren Berich einen scheibenförmigen Abschnitt aufweist, durch den ein rechteckiger Kanal diametral hindurch führt. Das Messer kann durch diesen Kanal und somit mitten durch das Branchengelenk geführt werden.

Die Ausbildung eines Drehgelenks zur schwenkbaren Lagerung einer oder zweier Branchen wird bei Gewebezangen oder bei Gewebescheren häufig mittels eines Querstifts bewirkt. Typische Beispiele dazu sind der US 2005/0113826 A1, der EP 1 958 583 A2, der WO 00/47124, der US 2011/0257680 A1, der US 2005/0159745 A1, der US 2002/0115997 A1, der GB 2 470 314 B, der US 8,394,094 B2, der US 6,113,598, der US 2011/0082494 A1, der US 2013/0085516 A1, der DE 10 2006 062 848 B4, der US 6,585,735 B1, der US 2002/0143358 A1, der US 2003/0216733 A1, der WO 2008/040483 A1 sowie der US 2004/0044363 A1 zu entnehmen.

Weiter ist es bekannt, zwei Branchen eines zangenartigen Instruments an voneinander beabstandeten Stellen jeweils mittels eines Querstifts zu lagern. Beispielhaft wird dazu auf die CN 101779979 B sowie auf die EP 2 845 548 B1 verwiesen. Die beiden beweglich gelagerten Branchen sind dabei an einem Sockel jeweils individuell schwenkbar gelagert, wobei zwischen den beiden Lagerstellen ein Abstand vorhanden ist, durch den ein Messer in einen im geschlossenen Zustand zwischen den beiden Branchen definierten Schlitz einführbar ist.

Wird ein Querstift zur Lagerung einer oder beider Branchen genutzt, ist dieser Querstift weiteren im Bereich der Branchen zu nutzenden Elementen im Weg. Ein solches Element kann z.B. ein Messer sein, das axial in distaler Richtung verschiebbar im Bereich des Zangeninstruments zu lagern ist. Wird die Branchenlagerung hingegen aus der Querschnittsmitte heraus verlagert, so dass der Weg für ein Messer oder sonstiges Element frei ist, trägt dies zur Vergrößerung des Gesamtquerschnitts des Instruments bei, was einer Verschlankung des Instruments im Wege steht.

Die US 6,656,177 B2 beschreibt eine Kauterisierungszange mit einer schwenkbaren Branche, zu deren Lagerung zwei zueinander fluchtende Zapfen dienen. Diese sitzen in einem proximalen Ende einer festen Branche jeweils seitlich. Der jeweilige Durchmesser der beiden Zapfen ist gering, weswegen die Zapfen aus einem entsprechend scherfesten Material auszubilden sind.

Es ist Aufgabe der Erfindung, ein Konzept für eine Gewebezange anzugeben, das eine schlanke und zugleich robuste Bauweise ermöglicht. Außerdem soll eine einfache Fertigung möglich sein.

Diese Aufgabe wird mit der Gewebezange nach Anspruch 1 gelöst:
Das erfindungsgemäße Zangeninstrument weist zwei Branchen auf, von denen wenigstens eine schwenkbeweglich gehalten ist, so dass die Branchen zwischen einander Gewebe fassen und zusammendrücken können. Zur Lagerung der Branchen dient ein Sockel mit einer Lagerstruktur, die zwei Lagerelemente umfasst, die zueinander koaxial angeordnet sind und gemeinsam einen Lagerzapfen bilden. Die Stirnseiten der beiden Lagerelemente können einander berühren oder, wie es bevorzugt wird, zwischen einander einen Abstand festlegen.

Die bewegliche Branche erstreckt sich so zwischen beiden Lagerelementen hindurch, dass diese Lagerelemente in zwei Öffnungen fassen können, die an der zweiten Branche an zwei voneinander weg weisenden Seiten angebracht sind. Die beiden Öffnungen sind koaxial zueinander angeordnet und weisen vorzugsweise einen kreisförmigen Querschnitt auf. Ebenso sind die beiden Lagerelemente koaxial zueinander angeordnet und weisen vorzugsweise einen Kreisquerschnitt auf. Weiter vorzugsweise sind die zwei Lagerelemente als Zapfen, vorzugsweise als Zylinderzapfen ausgebildet.

Diese Lageranordnung bildet ein Gelenk, das in dem Sockel bezüglich seines Querschnitts etwa mittig angeordnet werden kann. Trotz dieser mittigen Anordnung ist die Möglichkeit gegeben, andere durch die Sockelmitte führende Elemente, wie zum Beispiel ein Schneidmesser oder dergleichen, vorzusehen. Unabhängig davon ist ein platzsparender robuster Lageraufbau geschaffen, der mit wenigen Bauteilen auskommt, die kostengünstig herzustellen und leicht zu montieren sind.

Besonders einfach ist die Anordnung zu montieren, wenn die Lagerstruktur, wie es erfindungsgemäß vorgesehen ist, an einem aus Kunststoff bestehenden Lagereinsatz ausgebildet ist, der mit dem Sockel verbindbar ist. Insbesondere eignet sich zur Verbindung mit dem Sockel eine formschlüssige Verbindung, indem der Lagereinsatz vorzugsweise quer zu der Schwenkachse in den Sockel eingeführt und mit diesem verrastet wird. Dazu können an dem Sockel und dem Lagereinsatz entsprechende Raststrukturen vorgesehen sein. Alternativ und/oder zusätzlich kann eine Verbindungsstruktur zur ortsfesten Lagerung des Lagereinsatzes in dem Sockel vorgesehen sein. Die Verbindungsstruktur umfasst vorzugsweise führend ineinander greifende Elemente deren Führungsrichtung quer zu der Schwenkachse orientiert ist. Beispielsweise können an dem Lagereinsatz Vorsprünge, zum Beispiel prismatische Vorsprünge, ausgebildet sein, denen entsprechende an dem Sockel ausgebildete Nuten zugeordnet sind. Diese Nuten sind vorzugsweise in den aufeinander zu weisenden Wandungen eines in dem Sockel ausgebildeten Aufnahmefachs angeordnet, das quer durch den Sockel hindurchführt, Es weist somit ein erstes Fenster auf, aus dem die zweite Branche ragen kann sowie ein zweites Fenster zur Einführung des Lagereinsatzes in das Aufnahmefach.

Der Lagereinsatz kann durch zwei zueinander spiegelsymmetrisch ausgebildete Formteile insbesondere Spritzgussteile ausgebildet sein, die zwischen einander die zweite Branche halten. Die beiden Formteile können untereinander außerdem durch einen Steg, eine Wand, einen Zapfen oder dergleichen verbunden sein und somit ein einteiliges Spritzgussteil bilden. Dies ergibt eine einfache Herstellung und eine einfache Montage. Jedes der beiden Formteile kann eine Platte umfassen, die in montiertem Zustand an einer Seite der Branche anliegt und von der ein Lagerelement in eine Öffnung der Branche ragt. Der Lagereinsatz kann dadurch eine Doppelfunktion erfüllen, er ist Teil der Schwenklagerung der Branche und er bewirkt eine sichere elektrische Isolierung der Branche gegen den Sockel, der elektrisch leitend mit der anderen Branche verbunden sein kann. Auf diese Weise lassen sich sicher elektrische Spannungen von mehreren 100 V zwischen den Branchen aufrechterhalten.

Als Kunststoff eignet sich insbesondere PEEK oder LCP. Auch kann PPA (Polyphtalamide), PPS (Polyphenylensulfid) oder PAI (Polyamidimid) Anwendung finden.

Bei dem erfindungsgemäßen Zangeninstrument ist die aus zwei Zapfen bestehende Lagerachse komplett aus Kunststoff gefertigt. Weil zwischen den Zapfen ein Durchgang verbleibt, ist dort Platz für die Durchführung von Messer oder Zuleitungen vorhanden. Die Zapfen können mit einem sehr großen Durchmesser ausgebildet werden, was die Aufnahme großer Lagerkräfte und somit die Erzeugung hoher Anpresskräfte zwischen den Branchen des Instruments gestattet. Z.B. können die Zapfen jeweils einen Durchmesser aufweisen, der mindestens so groß ist, die in Schwenkrichtung zu messende Höhe der schwenkbaren Branche. Dadurch wird eine große Zapfenquerschnittsfläche und somit eine hohe maximale Lagerreaktionskraft erreicht, ohne den Kunststoff-Zapfen hinsichtlich seiner Scherfestigkeit zu überfordern.

Die an dem Lagereinsatz ausgewählte Führungsstruktur kann Vorsprünge umfassen, die an zwei voneinander weg weisenden Seiten des Lagereinsatzes ausgebildet sind. Vorzugsweise weisen diese Vorsprünge eine spitzwinklige Ecke auf, die bei geschlossenem Zangeninstrument in Kraftrichtung weist. Zusätzlich kann die Oberseite jedes Vorsprungs leicht gerundet sein. Auf diese Weise kann ein sicherer Halt des Lagereinsatzes in dem Sockel unterstütz werden. Die Rundung der Oberfläche kann dazu genutzt werden, die Rasteinrichtung, die den Lagereinsatz in dem Sockel hält, beim Schließen der Branche zu entlasten, um einen sicheren Sitz des Lagereinsatzes sicher zu stellen. Diese Geometrie kann dazu genutzt werden, bei geschlossenen Maulteil durch die Lagerreaktionskraft eine Selbstverklemmung herbei zu führen, indem der Lagereinsatz in seinen Sitz gepresst wird.

Die Erfindung richtet sich gemäß Anspruch 15 auch auf ein Verfahren zur Bereitstellung eines Zangeninstruments nach einem der vorstehenden Ansprüche, bei dem die Lagerstruktur z.B. als Spritzgussteil zunächst in Gestalt eines gestreckten, flachen Teils bereitgestellt wird. Bei diesem sind beide Lagerteile und ein Verbindungssteg zunächst in einer gemeinsamen Ebene oder in einem stumpfen Winkel zueinander angeordnet.

Die Lagerteile werden danach unter Verbiegung oder Entfernung oder Unterbrechung des Verbindungsstegs in zwei zueinander parallele, voneinander beabstandete Ebenen überführt. Zwischen den Lagerteilen kann das mindestens eine zu lagernde Teil angeordnet und mit diesem in das Fach des Sockels eingesetzt werden.

Weitere Einzelheiten vorteilhafter Ausführungsformen der Erfindung sind Gegenstand der Zeichnung der Beschreibung oder von Ansprüchen. Es zeigen:
Figur 1 ein erfindungsgemäßes Zangeninstrument, in schematisierter perspektivischer Übersichtsdarstellung,
Figur 2 das distale Ende des erfindungsgemäßen Zangeninstruments, in perspektivischer Prinzipdarstellung,
Figur 3 Einzelteile des Zangeninstruments nach Figur 2, in perspektivischer Darstellung,
Figur 4 das Zangeninstrument nach Figur 2, in perspektivischer Ansicht seiner Unterseite,
Figur 5 eine abgewandelte Ausführungsform des erfindungsgemäßen Zangeninstruments, in perspektivischer Explosionsdarstellung,
Figur 6 eine skizzenhafte Darstellung der an dem Zangeninstrument angreifenden Kräfte,
Figur 7 eine Querschnittsdarstellung des Zangeninstruments, geschnitten im Bereich seiner Lagereinrichtung, vereinfacht,
Figur 8 eine weiter abgewandelte Ausführungsform des erfindungsgemäßen Zangeninstruments, in perspektivischer Explosionsdarstellung
Figur 9 ein Lagereinsatz für eine erfindungsgemäßes Zangeninstrument in Einbauposition, in perspektivischer Darstellung,
Figur 10 einen Lagereinsatz für eine erfindungsgemäßes Zangeninstrument, nach Herstellung in perspektivischer Darstellung mit Blick auf seine Außenseite,

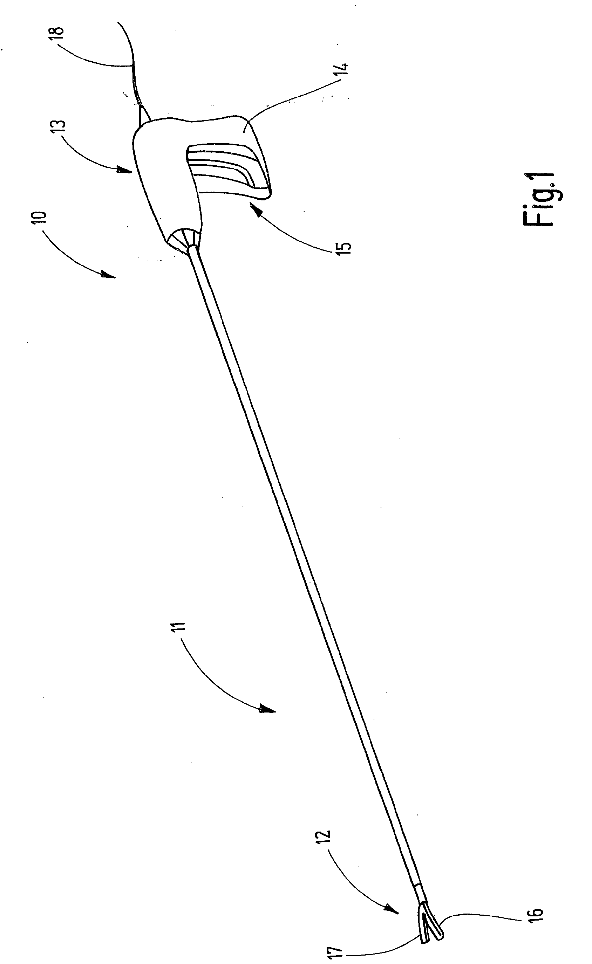

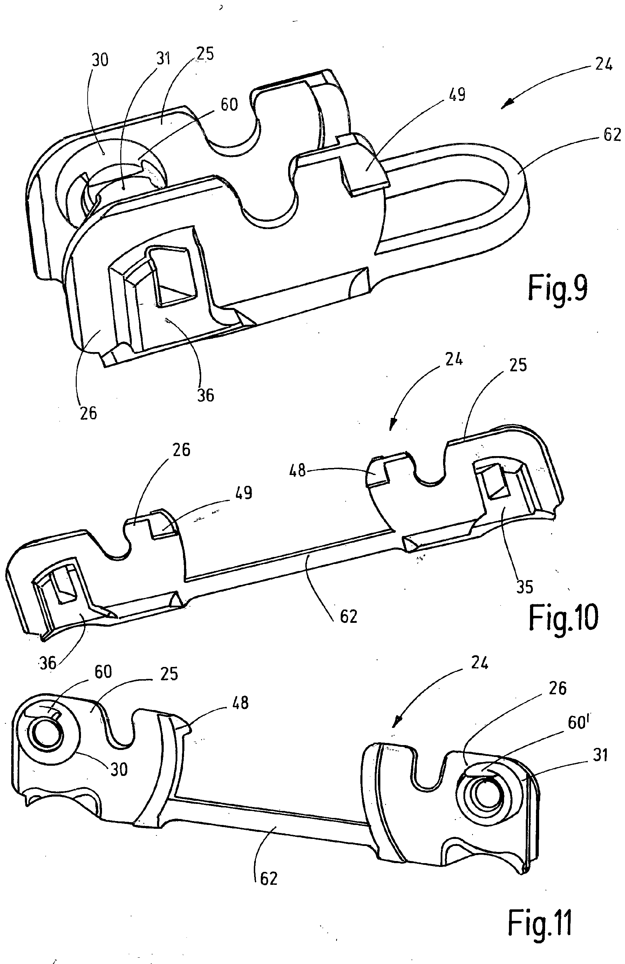

Figur 11 den Lagereinsatz nach Figur 10, in perspektivischer Darstellung mit Blick auf seine Innenseite. In Figur 1 ist ein Zangeninstrument 10 veranschaulicht, das hier beispielhaft als laparoskopisches Instrument mit einem Schaft 11 dargestellt ist, der endseitig eine Kauterisierungszange 12 trägt. Der Schaft 11 ist an seinem proximalen Ende von einem Gehäuse 13 getragen, das einen Griff 14 und ein Betätigungselement 15 aufweist. Letzteres dient zur Betätigung der Kauterisierungszange 12. Die Kauterisierungszange 12 weist zwei Branchen, nämlich eine erste Branche 16 und eine zweite Branche 17 auf, von denen mindestens ein beweglich gelagert ist und die im vorliegenden Ausführungsbeispiel mit HF-Strom beaufschlagbar sind, um zwischen ihnen gefasstes Gewebe zu koagulieren. Dazu ist das Zangeninstrument 10 über ein Kabel 18 mit einem nicht weiter veranschaulichten Generator verbunden.

Es wird darauf hingewiesen, dass anstelle des Zangeninstruments 10 im Rahmen der Erfindung auch andere Zangeninstrumente in Betracht zu ziehen sind, die zwei Branchen 16 und 17 aufweisen, von denen wenigstens eine gegen die andere beweglich gelagert ist. Das Anwendungsspektrum umfasst sowohl Instrumente für offenchirurgische Anwendungen mit oder ohne Schaft, laparoskopische Instrumente sowie Instrumente für den endoskopischen Einsatz. Zum Anwendungsspektrum gehören sowohl Instrumente mit elektrisch beaufschlagbaren Branchen 16, 17 sowie auch Instrumente mit Branchen, die lediglich eine mechanische Funktion zum Klemmen oder Schneiden haben und die nicht mit strombeaufschlagbar sind. Weiter gehören zum Anwendungsspektrum Instrumente mit einer Schneidvorrichtung, zum Beispiel einem Messer, das in oder an den Branchen 16, 17 zu bewegen ist, wie auch Instrumente ohne ein Messer. Zum Anwendungsspektrum der Erfindung gehören auch Zangeninstrumente mit feststehenden Messern, beispielsweise elektrisch beaufschlagbaren Schneidelektroden.

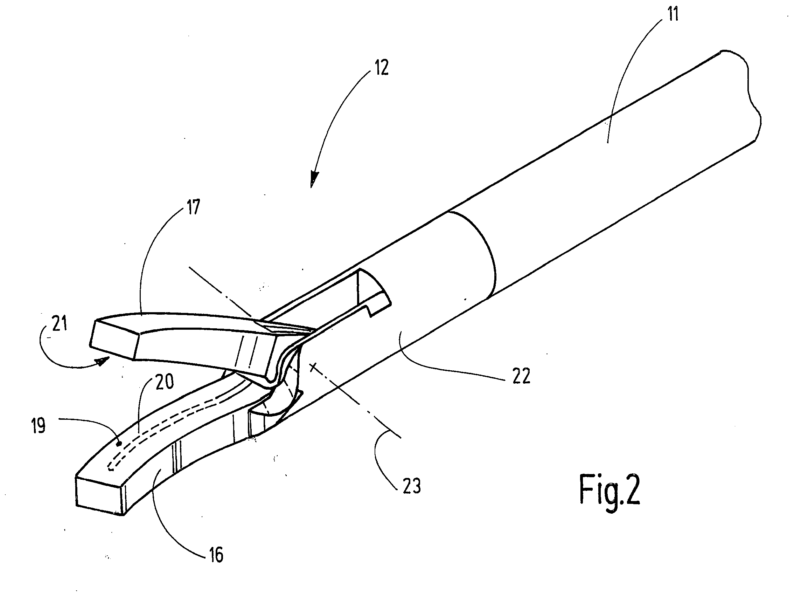

Die Kauterisierungszange 12 ist vergrößert in Figur 2 veranschaulicht. Die erste Branche 16 weist eine der zweiten Branche 17 zugewandte Gewebekontaktfläche 19 auf, die beispielsweise eben ausgebildet sein kann. Vorzugsweise ist die Gewebekontaktfläche 19 elektrisch leitend ausgebildet, um über das Kabel 18 mit elektrischer Spannung und Strom versorgt zu werden. Die Branche 16 kann gerade oder, wie dargestellt, leicht seitlich gekrümmt ausgebildet sein. Optional kann in der Gewebekontaktfläche 19 etwa mittig ein Längsschlitz 20 ausgebildet sein, wie er in Figur 2 gestrichelt veranschaulicht ist. Dieser kann der Führung eines nicht weiter veranschaulichten, axial beweglichen Schneidmessers dienen. Ebenso ist die zweite Branche 17 an ihrer der ersten Branche 16 zugewandten Seite mit einer Gewebekontaktfläche 21 versehen, die vorzugsweise eben ausgebildet ist. Die beiden Branchen 16, 17 sind vorzugsweise konturgleich, so dass sie mit beiden Gewebekontaktflächen 19, 21 passend aufeinander finden, wenn die zweite Branche 17 auf die erste Branche 16 geklappt wird. Auch die zweite Branche 17 kann mit einem Schlitz zur Messerführung versehen sein. Die Gewebekontaktfläche 21 ist vorzugsweise elektrisch leitfähig ausgebildet und beispielsweise über das Kabel 18 mit dem Generator verbindbar.

Wie Figur 2 weiter erkennen lässt, sind beide Branchen 16, 17 an einem Sockel 22 gehalten, der an dem distalen Ende des Schafts 11 angeordnet ist. Die erste Branche 16 ist dabei vorzugsweise fest mit dem Sockel 22 verbunden. Sie kann beispielsweise durch einen Fortsatz des Sockels 22 gebildet sein, so dass die erste Branche 16 und der Sockel 22 ein einstückiges Teil, zum Beispiel aus Kunststoff oder Keramik oder auch Metall bilden.

Die zweite Branche 17 ist um eine Schwenkachse 23 schwenkbar an dem Sockel 22 gelagert, die quer zu der Längsrichtung des Schafts 11 orientiert ist. Vorzugsweise liegt die Schwenkachse etwa auf der Mitte, d.h. auf dem Durchmesser des Sockelquerschnitts.

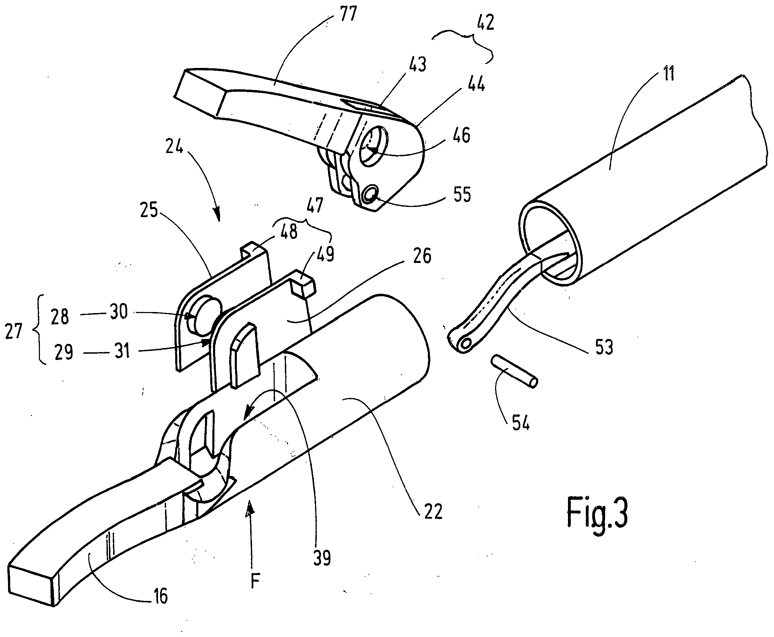

Zur schwenkbaren Lagerung der zweiten Branche 17 dient, wie insbesondere Figur 3 erkennen lässt, ein Lagereinsatz 24, der ein- oder zweiteilig ausgebildet sein kann und z.B. zwei zueinander spiegelsymmetrisch aufgebaute (oder auch unterschiedlich gestaltete) Lagerteile 25, 26 umfasst. Diese können als separate Teile bereitgestellt werden oder durch einen in Figur 3 verdeckten, zum Beispiel am rechten unteren Ende angeordneten Steg miteinander verbunden sein. Die Verbindung ist dabei so gestaltet, dass die beiden Lagerteile 25, 26 etwas aufeinander zu und voneinander weg federn können.

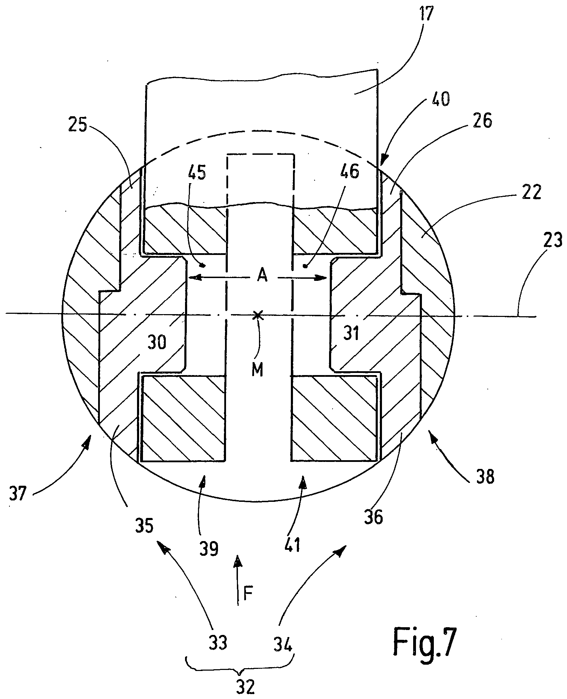

Der Lagereinsatz 24 ist vorzugsweise ein Kunststoffspritzgussteil, zu dem die beiden Lagerteile 25, 26 gehören und das von den Branchen 16, 17 gesondert ausgebildet und bereitgestellt wird. Es ist jedoch auch möglich, den Lagereinsatz an einer der Branchen 16, 17 mittels Spritzguss direkt anzubringen. Beide Lagerteile 25, 26 bestehen jeweils vorzugsweise aus einem plattenförmigen Grundabschnitt, an dem weitere Strukturen ausgebildet sind, insbesondere ist auf diese Weise eine Lagerstruktur 27 ausgebildet, zu der zwei Lagerelemente 28, 29 gehören. Das Lagerelement 28 wird vorzugsweise durch einen Zylinderzapfen 30 gebildet, der sich von einer Flachseite des Lagerteils 25 zu dem anderen Lagerteil 26 hin erstreckt. Ebenso wird das Lagerelement 29 vorzugsweise durch einen Zapfen 31 gebildet, der sich von dem Lagerteil 26 zu dem Lagerteil 25 hin erstreckt. Die beiden Zapfen 30, 31 sind vorzugsweise Zylinderzapfen, die zwischen einander einen Abstand A einschließen, der insbesondere auch aus Figur 7 ersichtlich ist. Allerdings ist es auch möglich, die Länge der Zapfen 30, 31 so zu gestalten, dass sie sich mit ihren Stirnseiten oder mit an den Stirnseiten angebrachten Vorsprüngen berühren. Die Durchmesser der Zapfen liegen vorzugsweise im Bereich zwischen 1 mm und 3 mm. Dieser relativ große Durchmesser gestattet die Ausbildung des Lagereinsatzes 24 aus Kunststoff. Gleichzeitig wird eine besonders schlanke Gestaltung des Zangenwerkzeugs ermöglicht, bei dem der Gelenkbereich einen relativ geringen Durchmesser von z.B. lediglich 5 mm - 5,7 mm aufweist.

Die Lagerteile 25, 26 sind an ihren voneinander weg weisenden Seiten mit Führungselementen 33, 34 in Gestalt von Vorsprüngen 35, 36 versehen, die zu einer Verbindungsstruktur 32 gehören. Diese Vorsprünge 35, 36 sind vorzugsweise parallelflankig begrenzte, in Draufsicht rechteckige oder trapezförmige prismatische Vorsprünge, die in einer Richtung quer zu der Schwenkachse 23 in entsprechende an dem Sockel 22 ausgebildete Nuten 37, 38 passen. Die Nuten 37, 38 sind an den aufeinander zu weisenden Wänden eines Fachs 39 ausgebildet (Figur 3 in Verbindung mit Figur 7), in das der Lagereinsatz 24 quer zu der Schwenkachse 23 einschiebbar ist. Dazu weist das Fach 39 ein oberes Fenster 40, aus dem sich die zweite Branche 17 heraus erstreckt, und ein unteres Fenster 41 auf, durch das der Lagereinsatz 24 in das Fach 39 einsetzbar ist. Das Fach 29 ist somit eine in Draufsicht ungefähr rechteckige Durchgangsöffnung durch den Sockel 22. Die zueinander parallelen Flanken der beiden Vorsprünge 35, 36 sind quer zu der Schwenkachse 23 orientiert und legen somit in Verbindung mit den Nuten 37, 38 eine Führungsrichtung F fest, die quer zu der Schwenkachse 23 und zugleich quer zur Längsachse des Schafts 11 steht. Diese Führungsrichtung F ist in Figur 3 und 7 jeweils lediglich zur Verdeutlichung durch einen Pfeil markiert. Andere Führungsrichtungen sind möglich.

Die zweite Branche 17 weist an ihrem proximalen Ende einen Scharnierabschnitt 42 auf, der zwei zueinander parallele Wandabschnitte 43, 44 umfassen kann, die beiden zueinander parallelen Wandabschnitte 43, 44 sind jeweils mit einer Öffnung 45, 46 versehen, wobei die Öffnungen 45, 46 vorzugsweise koaxial zueinander angeordnet sein können. Der Durchmesser der beiden Öffnungen 45, 46 kann dabei geringfügig größer sein als der Durchmesser der beiden vorzugsweise zylindrischen Zapfen 30, 31. Dabei ist die Anordnung der Zapfen 30, 31 und der Öffnungen 45, 46 vorzugsweise so getroffen, dass der Mittelpunkt M des Querschnitts des Sockels 21 innerhalb eines von den Öffnungen 45, 46 festgelegten Zylinders liegt. Bei dem Beispiel in Figur 7 liegt der Mittelpunkt M etwa auf der Schwenkachse 23, was jedoch insoweit nicht zwingend ist. Auch bei einem Versatz des Mittelpunkts M gegen die Schwenkachse 23 innerhalb der oben genannten Bedingung (Mittelpunkt M liegt innerhalb des von den Öffnungen 45, 46 definierten Zylinders) lässt sich eine schlanke und robuste leicht zu montierende Bauform für die Schwenklagerung der Branche 17 erreichen.

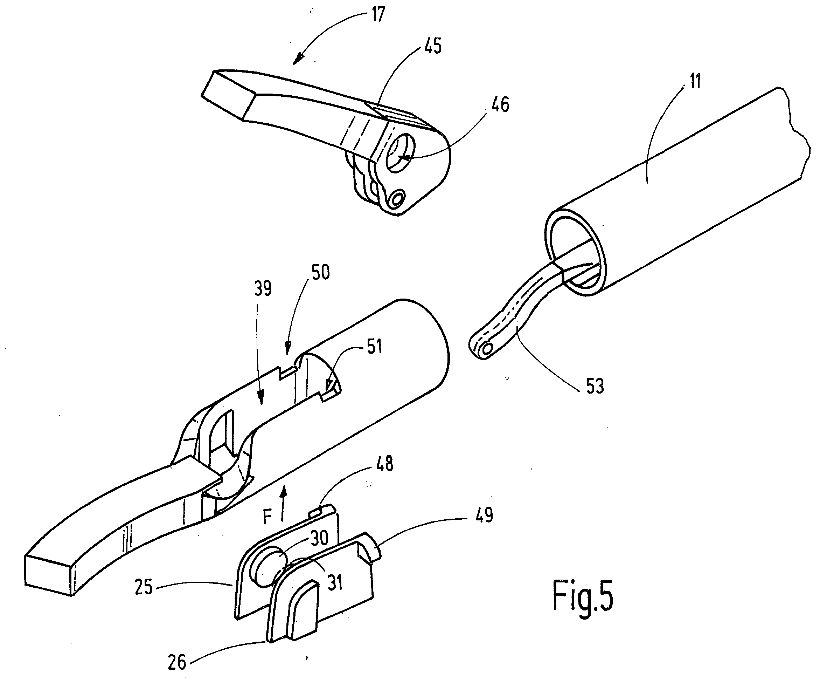

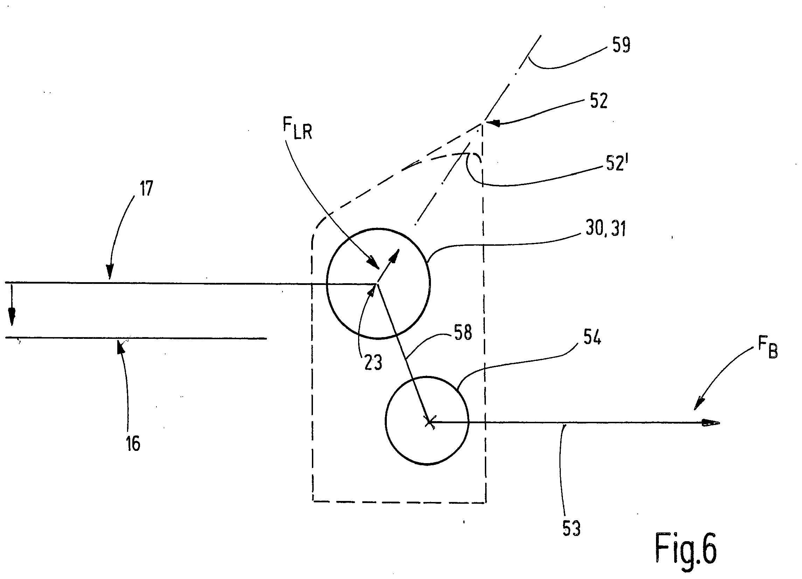

Die Verbindungsstruktur 32 kann als Rasteinrichtung ausgebildet sein, indem an den Vorsprüngen 35, 36 weitere Vorsprünge, beispielsweise Rastvorsprünge ausgebildet sind, die in Vertiefungen der Nuten 37, 38 greifen. Alternativ können als Rasteinrichtung 47 Rastvorsprünge 48, 49 an anderen Stellen der Lagerteile 25, 26 angeordnet sein, wie es Figur 3 prinzipiell veranschaulicht. Alternativ können den Rastvorsprüngen 48, 49, wie es Figur 5 veranschaulicht, entsprechende Rastausnehmungen 50, 51 zugeordnet sein, die zum Beispiel am oberen Rand des Fachs 39 ausgebildet sind. Zur Montage wird die Branche 17 zunächst so zwischen den Lagerteilen 25, 26 angeordnet, dass deren Zapfen 30, 31 in die Öffnungen 45, 46 fassen. Danach werden die Lagerteile 25, 26 federnd zusammengedrückt und diese Anordnung wird von unten her in Richtung F in das Fach 39 eingeführt, bis die Rastvorsprünge 48, 49 in die Ausnehmungen 50, 51 einrasten. Dabei fahren die Vorsprünge 35, 36 in den Nuten 37, 38 in Führungsrichtung F ein, bis sie mit ihren jeweiligen oberen Ecken in entsprechende vorzugsweise spitzwinklige Ecken der zugeordneten Nuten 37, 38 treffen. Figur 6 veranschaulicht dazu eine solche obere Ecke 52 am gestrichelt dargestellten Umriss einer Nut 37, 38. Die gesrichelte Linie 52' veranschaulicht dabei die zumindest vorzugsweise gerundete obere Seite oder Fläche des Vorsprungs 35 und/oder 36.

Der Branche 17 ist eine Betätigungsstange 53 zugeordnet, wie sie aus den Figuren 3 und 5 ersichtlich ist, Diese Betätigungsspanne ist beispielsweise über einen Querzapfen 54 an die Branche 17 angeschlossen, die dazu eine entsprechende Kupplungsöffnung 55 aufweist.

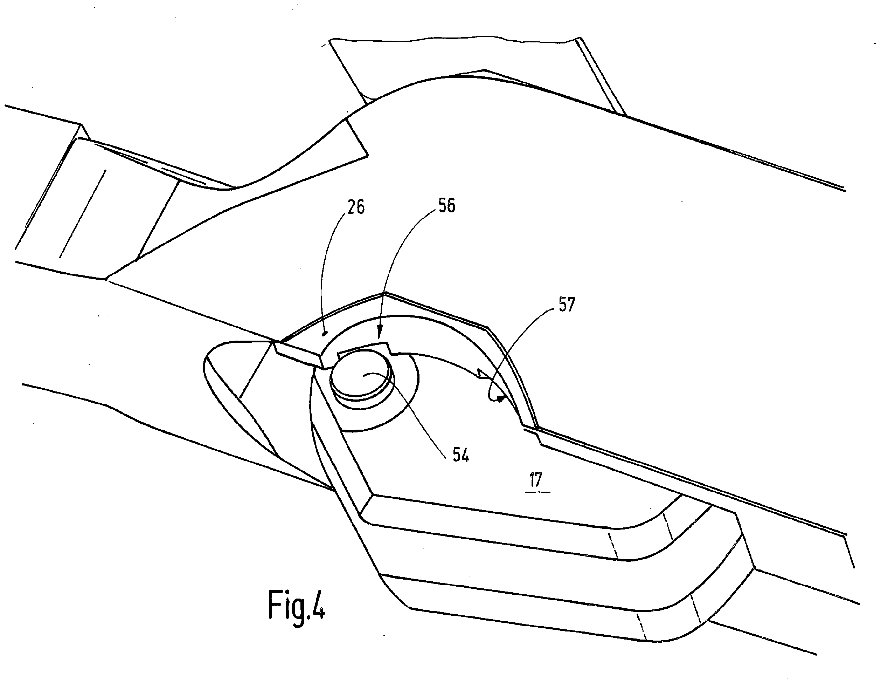

Wie Figur 4 erkennen lässt, kann der Stift 54 mit seinen beiden Enden jeweils die Außenfläche der Branche 17 etwas überragen, wie in Figur 4 an dem dem Betrachter zugewandten Ende des Stifts 54 dargestellt ist. Das Lagerteil 26 (wie auch das in Figur 4 verdeckte Lagerteil 25) kann für das Ende des Stifts 54 in beiden Endlagen der Branche 17, d.h. in voll geöffnetem wie in voll geschlossenem Zustand jeweils eine Tasche 56, 57 aufweisen, die das Ende des Stifts 54 aufnimmt und diesen bezüglich seiner Längsrichtung (die parallel zu der Schwenkachse 23 ist) sichert. Damit ist ein versehentlicher Verlust des Stifts 54 in Betrieb nicht möglich, während Montage und Demontage desselben in einer Zwischenstellung der Branche 17 zwischen der ganz geschlossenen und ganz offenen Stellung leicht möglich ist.

Zur Funktion und Kinematik des Zangeninstruments 10 wird auf Figur 6 verwiesen, in der neben der schon angesprochenen gestrichelt dargestellten Kontur des Führungselements 33 und 34 die gemeinsame Kreiskontur der Zapfen 33, 31 veranschaulicht ist, die konzentrisch zu der Schwenkachse 23 sind. Damit ergibt sich bezüglich des Mittelpunkts des Stifts 54 ein Hebelarm 58, an dem die Betätigungsstange 53 mit einer Betätigungskraft F_{B} ziehend angreifen kann. Die in Figur 6 lediglich schematisch veranschaulichte Branche 17 wird dadurch in Richtung auf die Branche 16 gespannt. Dabei treten an der durch die Zapfen 30, 31 gebildeten Lagereinrichtung Lagerreaktionskräfte auf F_{LR} auf, deren Richtung in Figur 6 durch einen Pfeil und eine in Richtung des Pfeils liegende strichpunktierte Linie 59 angedeutet ist. Diese Lagerreaktionskraft F_{LR} zeigt vorzugsweise wenigstens ungefähr in Richtung der Ecke 52, so dass die strichpunktierte Line 59 durch diese Ecke geht. Damit kann insgesamt ein Festsetzen des Lagereinsatzes 24 und eine Entlastung der Rasteinrichtung 47 erreicht werden so dass auch bei großer Kraftbeaufschlagung der Branchen 16, 17 sichergestellt ist, dass der Lagereinsatz 24 ordnungsgemäß in seiner Montageposition bleibt.

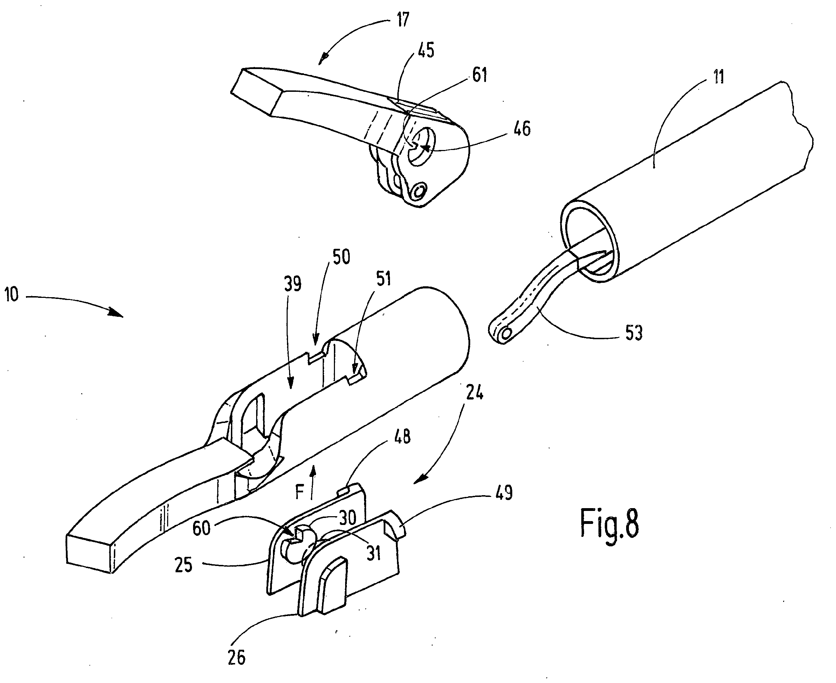

In Figur 8 ist eine weitere Ausführungsform des Zangeninstruments 10 veranschaulicht, zu dessen Aufbau und Funktion zunächst auf die vorstehende Beschreibung, dabei insbesondere die Beschreibung der Ausführungsform nach Figur 5 verwiesen wird. Bei dem Zangeninstrument 10 nach Figur 8 ist zwischen dem Lagereinsatz 24 und der Branche 17 eine Drehwinkelbegrenzungseinrichtung 60 wirksam, die hier beispielhaft durch eine in wenigstens einem der Zapfen 30, 31 vorgesehene Ausnehmung gebildet ist. Dieser Ausnehmung ist ein an dem Rand der Öffnung 45, 46 vorgesehener Vorsprung 61 zugeordnet, der in Umfangrichtung des Zapfens 30, 31 in der Ausnehmung begrenz beweglich ist. Dadurch wird der maximale Schwenkwinkel der Branche 17 begrenzt.

In Figur 9 ist eine abgewandelte Ausführungsform eines Lagereinsatzes 24 veranschaulicht, der beispielsweise als Spritzgussteil ausgebildet sein kann. Elemente dieses Lagereinsatzes 24, die mit vorbeschriebenen Lagereinsätzen funktions- und/oder strukturgleiche sind, sind in Figur 9 mit bereits eingeführten Bezugszeichen bezeichnet. Die Beschreibung der Ausführungsformen nach Figur 1 bis 8 gilt für den Lagereinsatz 24 nach Figur 9 entsprechend. Der Lagereinsatz 24 ist in Einbauposition dargestellt, in der die Lagerteile 25, 26 nebeneinander in voneinander beabstandeten Ebenen angeordnet sind. Ein die beiden Lagerteile 25, 26 einstückig miteinander verbindender Steg 62 ist unter elastischer und/oder plastischer Verformung u-förmig gebogen.

Figur 10 und 11 veranschaulichen den Lagereinsatz 24 in gestreckter Position nach der Herstellung, z.B. direkt nach dem Ausformen aus einer Spritzgussform. Der Lagereinsatz ist in dieser Form gut handhabbar und aufbewahrbar. Vor dem Einbau in ein Zangeninstrument, wird er in die Form nach Figur 9 überführt. Als Drehwinkelbegrenzungseinrichtung 60, 60' kann der Zapfen 30, 31 an seiner Stirnseite eine Nase aufweisen. Außerdem kann der Zapfen 31, 31 hohl ausgebildet sein und/oder weitere Strukturen aufweisen.

Die Vorsprünge 35, 36 können massiv ausgebildet oder an ihrer Außenseite mit einer oder mehreren Ausnehmungen versehen sein. Diese Ausnehmungen können z.B. als Nuten gestaltet sein, um eine Verzahnung mit Strukturen in dem Fach 39 zu erreichen.

Bei dem erfindungsgemäßen Zangeninstrument 10 ist zur Lagerung mindestens einer schwenkbar gelagerten Branche 17 ein Lagereinsatz 24 vorgesehen, der in einen Sockel 22 eingefügt ist, um die wenigstens eine schwenkbare Branche 17 zu lagern. Der Lagereinsatz 24 wird dazu z.B. quer zur Schwenkachse 23 in ein entsprechendes Fach 39 des Sockels 22 eingefügt und dort in dem Fach 39, beispielsweise durch Rastwirkung, befestigt. Der Lagereinsatz 24 weist zwei Lagerelemente 28, 29, beispielsweise in Gestalt zylindrischer Zapfen 30, 31 auf, die von zueinander parallel orientierten beispielsweise plattenförmigen Lagerteilen 25, 26 des Lagereinsatzes 24 aufeinander zuweisend angeordnet sind, ohne einander zu berühren. Die beiden Zapfen 30, 31 greifen in entsprechende Öffnungen 45, 46 der Branche 17, wobei der zwischen den Stirnseiten der Zapfen 30, 31 verbleibende Abstand A zur Anordnung sonstiger Elemente, wie beispielsweise eines Schneidmessers, genutzt werden kann.

### Bezugszeichen:

- 10: Zangeninstrument
- 11: Schaft
- 12: Kauterisierungszange
- 13: Gehäuse
- 14: Griff
- 15: Betätigungselement
- 16: erste Branche
- 17: zweite Branche
- 18: Kabel
- 19: Gewebekontaktfläche
- 20: Längsschlitz
- 21: Gewebekontaktfläche
- 22: Sockel
- 23: Schwenkachse
- 24: Lagereinsatz
- 25, 26: Lagerteile
- 27: Lagerstruktur
- 28, 29: Lagerelemente
- 30, 31: Zapfen
- A: Abstand der Stirnseiten der Zapfen 30, 31
- 32: Verbindungsstruktur
- 33, 34: Führungselemente
- 35, 36: Vorsprünge
- 37, 38: Nuten
- 39: Fach zur Aufnahme des Lagereinsatzes
- 40: oberes Fenster
- 41: unteres Fenster
- 42: Scharnierabschnitt
- 43, 44: Wandabschnitt
- 45, 46: Öffnung
- M: Querschnittsmitte des zylindrischen Sockels 22
- 47: Rasteinrichtung
- 48, 49: Rastvorsprünge
- 50, 51: Ausnehmungen
- 52: Ecke
- 52': obere Seite des Vorsprungs 35, 36
- 53: Betätigungsstange
- 54: Stift
- 55: Kupplungsöffnung
- 56, 57: Taschen zur Sicherung des Stifts 54
- 58: Hebelarm
- F_{B}: Betätigungskraft
- F_{LR}: Lagerreaktionskraft
- 59: Strichpunktlinie
- 60, 60': Drehwinkelbegrenzungseinrichtung
- 61: Vorsprung
- 62: Steg

## Patentansprüche

1. Zangeninstrument (10) zum Behandeln von biologischem Gewebe,
mit einer ersten Branche (16), die an einem Sockel (22) gehalten ist und sich von diesem weg erstreckt, mit einer zweiten Branche (17), die an dem Sockel (22) um eine Schwenkachse (23) schwenkbeweglich gehalten ist,
wobei zur schwenkbaren Lagerung der zweiten Branche (17) eine Lagerstruktur (27) mit zwei Lagerelementen (28, 29) vorgesehen ist, die koaxial zueinander angeordnet sind und gemeinsam einen geteilten Lagerzapfen bilden,
**dadurch gekennzeichnet, dass**
die Lagerstruktur (27) an einem aus einem Kunststoff bestehenden Lagereinsatz (24) ausgebildet ist, der mit dem Sockel (22) verbunden ist.

2. Zangeninstrument nach Anspruch 1, **dadurch gekennzeichnet, dass** der Lagereinsatz (24) und eine Verbindungsstruktur (32) zur ortsfesten Lagerung des Lagereinsatzes (24) in dem Sockel (22) aufweist.

3. Zangeninstrument nach Anspruch 1 oder 2, **dadurch gekennzeichnet, dass** der Lagereinsatz (24) quer zu der Schwenkachse (23) in den Sockel (22) einführbar ist.

4. Zangeninstrument nach Anspruch 1, **dadurch gekennzeichnet, dass** der Sockel (22) ein Aufnahmefach (39) für den Lagereinsatz (24) aufweist, wobei der Sockel (22) ein erstes Fenster (40) für die zweite Branche (17) und ein zweites Fenster (41) zur Einführung des Lagereinsatzes (24) in das Aufnahmefach (39) aufweist.

5. Zangeninstrument nach einem der vorstehenden Ansprüche, **dadurch gekennzeichnet, dass** der Lagereinsatz (24) zwei zueinander spiegelsymmetrisch ausgebildete Lagerteile (25, 26) umfasst, an denen die Lagerstruktur (27) und die Verbindungsstruktur (32) ausgebildet ist.

6. Zangeninstrument nach einem der vorstehenden Ansprüche, **dadurch gekennzeichnet, dass** die zwei zueinander koaxial angeordneten Lagerelemente (28, 29) aufeinander zu weisende Zapfen (30, 31) sind, die zwischen einander einen Abstand (A) festlegen.

7. Zangeninstrument nach einem der vorstehenden Ansprüche, **dadurch gekennzeichnet, dass** die zweite Branche (17) an zwei voneinander weg weisenden Seiten zwei zueinander koaxial angeordnete Öffnungen (45, 46) aufweist, die als Drehlager dienen.

8. Zangeninstrument nach einem der vorstehenden Ansprüche, **dadurch gekennzeichnet, dass** die zwei zueinander koaxial angeordneten Lagerelemente (28, 29) zylindrisch ausgebildet sind.

9. Zangeninstrument nach einem der vorstehenden Ansprüche, **dadurch gekennzeichnet, dass** die zwei zueinander koaxial angeordneten Lagerelemente (28, 29) eine Schwenkwinkelbegrenzungseinrichtung (60, 60') aufweisen.

10. Zangeninstrument nach einem der vorstehenden Ansprüche, **dadurch gekennzeichnet, dass** der Lagereinsatz (24) aus PEEK oder LCP oder auch: PPA, PPS, PAI besteht.

11. Zangeninstrument Anspruch 9, **dadurch gekennzeichnet, dass** die zweite Branche (17) an zwei voneinander weg weisenden Seiten zwei zueinander koaxial angeordnete kreisförmige Öffnungen (45, 46) aufweist, die jeweils einen radial nach innen gerichtete Vorsprung aufweisen, der dazu eingerichtet ist, mit der Drehwinkelbegrenzungseinrichtung (60, 60') zusammenzuwirken.

12. Zangeninstrument nach einem der vorstehenden Ansprüche, **dadurch gekennzeichnet, dass** der Lagereinsatz (24) an voneinander weg weisenden Seiten Führungselemente (33, 34) aufweist, die, zusammen mit einer dazu komplementären, in dem Sockel (22) ausgebildeten Struktur eine Führungsrichtung (F) festlegt, die quer zu der Schwenkachse (23) orientiert ist, wobei die Führungselemente (33, 34) vorzugsweise durch polygonal umgrenzte Vorsprünge gebildet sind.

13. Zangeninstrument nach Anspruch 12, **dadurch gekennzeichnet, dass** jeder Vorsprung eine spitzwinklige Ecke (52) aufweist, die bei geschlossenen Zangeninstrument (10) in einer Kraftrichtung (F_{LR}) weist, um durch ihr Geometrie bei geschlossenen Maulteil in einer Kraftrichtung zu einer Selbstverklemmung zu führen.

14. Zangeninstrument nach einem der vorstehenden Ansprüche, **dadurch gekennzeichnet, dass** zwischen dem Lagereinsatz (24) und dem Sockel (22) wirkend eine Rasteinrichtung vorgesehen ist.

15. Verfahren zur Bereitstellung eines Zangeninstruments nach einem der vorstehenden Ansprüche, **dadurch gekennzeichnet, dass** die Lagerstruktur (27)
zunächst in Gestalt eines gestreckten Teils bereitgestellt wird, bei dem beide Lagerteile (25, 26) und ein Verbindungssteg (62) in einer gemeinsamen Ebene angeordnet sind,
wonach die Lagerteile (25, 26) unter Verbiegung oder Entfernung oder Unterbrechung des Verbindungsstegs in zwei zueinander parallele, voneinander beabstandete Ebenen überführt werden,
wonach die Lagerstruktur (27) in das Fach (39) eingesetzt wird.

## Claims

1. Forceps instrument (10) for treating biological tissue,
with a first branch (16) which is held on a base (22) and extends away from this, with a second branch (17) which is held on the base (22) so as to be pivotable about a pivot axis (23),
wherein for pivotable mounting of the second branch (17), a bearing structure (27) is provided which has two bearing elements (28, 29) which are arranged coaxially to one another and together form a split bearing journal,
**characterised in that**
the bearing structure (27) is formed from a bearing insert (24) which consists of plastic and is connected to the base (22).

2. Forceps instrument according to claim 1, **characterised in that** the bearing insert (24) comprises a connecting structure (32) for stationary mounting of the bearing insert (24) in the base (22).

3. Forceps instrument according to claim 1 or 2, **characterised in that** the bearing insert (24) can be introduced into the base (22) transversely to the pivot axis (23).

4. Forceps instrument according to claim 1, **characterised in that** the base (22) has a receiving compartment (39) for the bearing insert (24), wherein the base (22) has a first window (40) for the second branch (17) and a second window (41) for insertion of the bearing insert (24) in the receiving compartment (39).

5. Forceps instrument according to any of the preceding claims, **characterised in that** the bearing insert (24) comprises two bearing parts (25, 26) which are mirror-symmetrical to one another and on which the bearing structure (27) and the connecting structure (32) are formed.

6. Forceps instrument according to any of the preceding claims, **characterised in that** the two mutually coaxially arranged bearing elements (28, 29) are pegs (30, 31) which point towards one another and define a distance (A) between them.

7. Forceps instrument according to any of the preceding claims, **characterised in that** on two sides facing away from one another, the second branch (17) has two openings (45, 46) which are arranged coaxially to one another and serve as pivot bearings.

8. Forceps instrument according to any of the preceding claims, **characterised in that** the two mutually coaxially arranged bearing elements (28, 29) are cylindrical.

9. Forceps instrument according to any of the preceding claims, **characterised in that** the two mutually coaxially arranged bearing elements (28, 29) have a pivot-angle-limiting device (60, 60').

10. Forceps instrument according to any of the preceding claims, **characterised in that** the bearing insert (24) consists of PEEK or LCP or also PPA, PPS, PAI.

11. Forceps instrument according to claim 9, **characterised in that** on two sides facing away from one another, the second branch (17) has two circular openings (45, 46) which are arranged coaxially to one another and each have a radially inwardly directed protrusion which is configured to cooperate with the rotation-angle-limiting device (60, 60').

12. Forceps instrument according to any of the preceding claims, **characterised in that** on sides facing away from one another, the bearing insert (24) has guide elements (33, 34) which, together with a complementary structure formed in the base (22), define a guide direction (F) oriented transversely to the pivot axis (23), wherein the guide elements (33, 34) are preferably formed by polygonally contoured protrusions.

13. Forceps instrument according to claim 12, **characterised in that** each protrusion has a acute-angled corner (52) which points in a force direction (F_{LR}) when the forceps instrument (10) is closed, in order, by its geometry, when the jaw part is closed, to lead to a self-clamping in one force direction.

14. Forceps instrument according to any of the preceding claims, **characterised in that** a catch device is provided which acts between the bearing insert (24) and the base (22).

15. Method for providing a forceps instrument according to any of the preceding claims, **characterised in that** initially, the bearing structure (27) is provided in the form of an elongate part, wherein the two bearing parts (25, 26) and a connecting web (62) are arranged in a common plane,
whereafter, by bending or removal or interruption of the connecting web, the bearing parts (25, 26) are transferred into two mutually parallel, spaced apart planes, whereafter the bearing structure (27) is inserted in the compartment (39).

## Revendications

1. Instrument à pince (10) destiné à traiter des tissus biologiques,
comprenant un premier mors (16) qui est tenu sur un socle (22) et s'étend à partir de celui-ci, comprenant un deuxième mors (17) qui est monté sur le socle (22) avec possibilité de pivotement autour d'un axe de pivotement (23),
sachant que pour le montage pivotant du deuxième mors (17), il est prévu une structure formant palier (27) comportant deux éléments de support (28, 29) qui sont disposés de façon coaxiale l'un avec l'autre et forment ensemble un tourillon de palier divisé,
**caractérisé en ce que**
la structure formant palier (27) est réalisée sur un insert de palier (24) qui est constitué de matière plastique et est relié au socle (22).

2. Instrument à pince selon la revendication 1, **caractérisé en ce que** l'insert de palier (24) et une structure de liaison (32) présente pour le support stationnaire de l'insert de palier (24) dans le socle (22).

3. Instrument à pince selon la revendication 1 ou 2, **caractérisé en ce que** l'insert de palier (24) peut être introduit dans le socle (22) transversalement à l'axe de pivotement (23).

4. Instrument à pince selon la revendication 1, **caractérisé en ce que** le socle (22) présente un compartiment (39) recevant l'insert de palier (24), le socle (22) étant doté d'une première fenêtre (40) pour le deuxième mors (17) et d'une deuxième fenêtre (41) destinée à introduire l'insert de palier (24) dans le compartiment de réception (39).

5. Instrument à pince selon l'une des revendications précédentes, **caractérisé en ce que** l'insert de palier (24) comprend deux parties de palier (25, 26) qui sont réalisées avec une symétrie spéculaire l'une par rapport à l'autre et sur lesquelles sont réalisées la structure formant palier (27) et la structure de liaison (32).

6. Instrument à pince selon l'une des revendications précédentes, **caractérisé en ce que** les deux éléments de palier (28, 29) disposés de façon coaxiale l'un avec l'autre sont des tourillons (30, 31) qui sont dirigés l'un vers l'autre et définissent une distance (A) entre eux.

7. Instrument à pince selon l'une des revendications précédentes, **caractérisé en ce que** le deuxième mors (17) présente, sur deux côtés tournés dans des sens opposés, deux ouvertures (45, 46) qui sont disposées de façon coaxiale l'une avec l'autre et servent de palier de rotation.

8. Instrument à pince selon l'une des revendications précédentes, **caractérisé en ce que** les deux éléments de palier (28, 29) disposés de façon coaxiale l'un avec l'autre sont réalisés sous une forme cylindrique.

9. Instrument à pince selon l'une des revendications précédentes, **caractérisé en ce que** les deux éléments de palier (28, 29) disposés coaxialement l'un avec l'autre présentent un dispositif de limitation d'angle de pivotement (60, 60').

10. Instrument à pince selon l'une des revendications précédentes, **caractérisé en ce que** l'insert de palier (24) est constitué de PEEK ou de LCP ou bien également : de PPA, PPS, PAI.

11. Instrument à pince selon la revendication 9, **caractérisé en ce que** le deuxième mors (17) présente, sur deux côtés tournés dans des sens opposés, deux ouvertures (45, 46) circulaires qui sont disposées de façon coaxiale l'une avec l'autre et présentent chacune une saillie tournée radialement vers l'intérieur et conçue pour coopérer avec le dispositif de limitation d'angle de rotation (60, 60').

12. Instrument à pince selon l'une des revendications précédentes, **caractérisé en ce que** l'insert de palier (24) présente, sur des côtés tournés dans des sens opposés, des éléments de guidage (33, 34) qui, conjointement avec une structure complémentaire desdits éléments, réalisée dans le socle (22), définit une direction de guidage (F) qui est orientée transversalement à l'axe de pivotement (23), les éléments de guidage (33, 34) étant de préférence constitués de saillies délimitées sous une forme polygonale.

13. Instrument à pince selon la revendication 12, **caractérisé en ce que** chaque saillie présente un coin (52) à angle aigu qui, lorsque l'instrument à pince (10) est fermé, est orienté dans une direction de force (F_{LR}) afin de provoquer de par sa géométrie un autoblocage dans une direction de la force lorsque la partie formant fourche est fermée.

14. Instrument à pince selon l'une des revendications précédentes, **caractérisé en ce qu'**il est prévu un dispositif d'encliquetage qui agit entre l'insert de palier (24) et le socle (22).

15. Procédé de mise à disposition d'un instrument à pince selon l'une des revendications précédentes, **caractérisé en ce que** la structure formant palier (27)
est d'abord mise à disposition sous la forme d'une pièce allongée dans laquelle les deux parties de palier (25, 26) et une tige de liaison (62) sont disposées dans un plan commun,
à la suite de quoi les parties de palier (25, 26), par déformation ou retrait ou interruption de la tige de liaison, sont amenées dans deux plans mutuellement parallèles et espacés l'un de l'autre,
à la suite de quoi la structure formant palier (27) est mise en place dans le compartiment (39).
